(19) Europäisches Patentamt
European Patent Office
Office européen des brevets

(11) Veröffentlichungsnummer: **0 418 584 B1**

(12) # EUROPÄISCHE PATENTSCHRIFT

(45) Veröffentlichungstag der Patentschrift: **15.06.94**

(51) Int. Cl.5: **C12N 9/04**, C12N 9/96, C12Q 1/32

(21) Anmeldenummer: **90116169.5**

(22) Anmeldetag: **23.08.90**

(54) **D-Malat-Bestimmung.**

(30) Priorität: **25.08.89 DE 3928209**

(43) Veröffentlichungstag der Anmeldung:
**27.03.91 Patentblatt 91/13**

(45) Bekanntmachung des Hinweises auf die Patenterteilung:
**15.06.94 Patentblatt 94/24**

(84) Benannte Vertragsstaaten:
**AT BE CH DE DK ES FR GB GR IT LI LU NL SE**

(56) Entgegenhaltungen:
**EP-A- 0 089 640**

**NATURE, Band 212, Nr. 5070, 31. Dezember 1966, Seiten 1611-1612, Londen, GB; J.R. STERN et al.: "Inducible D-malic enzyme in Escherichia coli"**

**ACTA CHEMICA SCANDINAVICA, Band B34, 1980, Seiten 423-427, Acta Chemica Scandinavica; M. LÄHDESMÄKI et al.: "D-Malate dehydrogenase from Pseudomonas fluorescens UK-1"**

(73) Patentinhaber: **BOEHRINGER MANNHEIM GMBH**

**D-68298 Mannheim(DE)**

(72) Erfinder: **Beutler, Hans-Otto, Dr.**
**Zugspitzstrasse 28**
**D-8132 Tutzing(DE)**
Erfinder: **Lang, Gunter**
**Lange Strasse 22**
**D-8132 Tutzing(DE)**
Erfinder: **Kaluza, Klaus, Dr.**
**Antdorfer Strasse 12**
**D-8121 Habach(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**DEUTSCHE LEBENSMITTEL-RUNDSCHAU, Band 86, Nr. 11, 1990, Seiten 341-344; H.O.BEUTLER et al.: "A new method for the enzymatic determination of D-malic acidin foodstuffs"**

**Beschreibung**

Zur Bestimmung von D-Malat (D-Äpfelsäure) in einer wäßrigen Lösung kann die NAD-abhängige D-Malat-Dehydrogenase decarboxylierend [E.C. 1.1.1.83] (D-MDH, D-MalicEnzym) eingesetzt werden. Das Enzym katalysiert folgende Reaktion:

$$\text{D-Malat} + NAD^{+} \xrightarrow[Mg^{2+};\ Mn^{2+}]{\text{D-MDH}} \text{Pyruvat} + CO_2 + NADH + H^{+} \qquad (1)$$

Ein D-Malic-Enzym aus E. coli K12 (Crookes Stamm) wurde von Stern und Hegre (Nature 212 (1966), 1611-1612) beschrieben. Die enzymatische Aktivität dieses D-Malic-Enzyms wurde nur im Rohextrakt getestet. Es katalysiert eine Reaktion gemäß Gleichung (1), also die Umsetzung von D-Malat zu Pyruvat in Gegenwart von $NAD^+$ und $Mg^{2+}$-bzw. $M^{2+}$.-Ionen. Für die enzymatische Aktivität ist die Anwesenheit von $K^+$, $NH_4{}^+$ oder $Rb^+$-Ionen erforderlich. Durch Zugabe von EDTA (3,3 µmol/ml) wird die Reaktion inhibiert. Ist $NADP^+$ anstelle von $NAD^+$ im Reaktionsgemisch anwesend, so wird eine geringe Umsetzung von D-Malat zu Pyruvat und $CO_2$ (8 %), bezogen auf die Reaktionsrate, in Gegenwart von $NAD^+$ gemessen.

Ein weiteres D-Malic-Enzym aus Pseudomonas fluorescens wurde von Knichel und Radler (Eur. J. Biochem. 123 (1982), 547-552) beschrieben. Dieses D-Malic-Enzym wurde gereinigt und durch Zugabe von 50 mmol/l Ammoniumsulfat und 1 mmol/l EDTA stabilisiert. Es wurde ein Enzym mit einem Molekulargewicht von ungefähr 175 kD (SDS-Gel und Gelfiltration) und einer spezifischen Aktivität von 4 bis 5 U/mg Protein erhalten, das durch zweiwertige Kationen aktiviert wird. Die Aktivierung der enzymatischen Aktivität durch Mangan-Ionen ist nicht höher als die durch Magnesium-Ionen. Die Km-Werte betragen für Malat 0,3 mmol/l und für $NAD^+$ 0,08 mmol/l. Das pH-Optimum für dieses Enzym liegt im Bereich zwischen 8,1 und 8,8.

L-Malat ist die naturlich vorkommende Form der beiden Stereoisomere von Malat. Um auf illegale Weise den Säuregehalt von Fruchtsäften und Weinen zu erhöhen, kann synthetisches Malat (bestehend aus einem Racemat von D- und L-Malat) zugesetzt werden. Zur Erkennung eines möglichen D-Malat-Zusatzes ist eine genaue Bestimmung des D-Malat-Gehalts in Gegenwart eines großen Überschusses an L-Malat erforderlich.

Zur Messung des D-Malat-Gehalts sind bereits mehrere Verfahren bekannt. So kann in Fruchtsäften der Gesamtgehalt an Malat mit einer chemischen Methode (Rebelein, Deut. Lebensm. Rundschau 60 (1964) 140-144) erfaßt werden. Von diesem Wert wird der Gehalt an L-Malat, der enzymatisch bestimmt wurde (Methoden der enzymatischen Analyse, Band 2 (1970), 1544-1548) abgezogen. Die Differenz beider Werte liefert den Gehalt an D-Malat. Diese Messung ist unzuverlässig, weil der geringe D-Malat-Gehalt aus einer Differenz zweier großer Zahlen ermittelt wird. Außerdem werden bei diesem Testverfahren möglicherweise Ester und Lactone von L-Malat nicht erfaßt.

Eine zweite Methode zur Bestimmung des D-Malat-Gehalts in wäßrigen Lösungen beruht auf der Tatsache, daß jede synthetisch hergestellte Äpfelsäure (Racemat aus D- und L-Malat) stets 2 bis 5 % Fumarsäure enthält. Da in Wein nur vernachlässigbar geringe Mengen an Fumarsäure enthalten sind, bedeutet der Nachweis von Fumarsäure mittels HPLC, daß einer Probe synthetische Äpfelsäure, also auch D-Malat zugesetzt worden ist. Dieser Nachweis kann allerdings nur qualitativ sein, da der jeweilige Fumarsäure-Gehalt des Racemats aus D- und L-Malat nicht genau bekannt ist.

Zur Bestimmung des Gehalts von D-Malat kann auch ein D-Malic-Enzym eingesetzt werden. So haben Knichel und Radler (Z. Lebensm. Unters. Forsch. 174 (1982) 296-299) für das D-Malic-Enzym aus Pseudomonas fluorescens eine Methode zur enzymatischen Bestimmung von D-Malat entwickelt. Dieser Test hat jedoch nur geringe Bedeutung erlangt, weil das Pseudomonas-Enzym nur eine geringe Haltbarkeit (wenige Tage bei Lagerung bei 4°C) aufweist. Weiterhin wird die Messung des D-Malat-Gehalts in Fruchtsäften unter Verwendung des Pseudomonas-Enzyms durch einen hohen Anteil an Fremdaktivitäten erschwert.

Aufgabe der vorliegenden Erfindung ist es somit, ein D-Malic-Enzym zu isolieren, das analytisch rein zur Verwendung in einem D-Malat-Test hergestellt werden kann, eine hohe Spezifität für D-Malat aufweisen soll (keine störenden Fremdaktivitäten), eine hohe Stabilität besitzen soll und in einem schnell und einfach auszuführenden D-Malat-Test eingesetzt werden kann.

Ein Gegenstand der Erfindung ist eine $NAD^+$-abhängige D-Malat-Dehydrogenase, decarboxylierend, (E.C. 1.1.1.83) aus E. coli, für deren Aktivität keine Anwesenheit von $K^+$, $Rb^+$ oder $NH_4{}^+$-Ionen erforderlich ist, daß seine Aktivität nicht durch EDTA (0,7 mg/ml Testlösung) gehemmt wird, daß die Umsetzung von D-Malat zu Pyruvat und $CO_2$ bei Anwesenheit von $NADP^+$ weniger als 0,5 % der Umsetzung bei Anwesenheit

von $NAD^+$ beträgt, daß es aus zwei Untereinheiten mit jeweils etwa 42 kD Molekulargewicht (SDS-Gel) besteht und daß es aus E.coli DSM 5496 erhältlich ist.

Die Aktivität dieses Enzyms wird durch $Mg^{2+}$-, aber noch mehr durch $Mn^{2+}$-Ionen gesteigert. Der Km-Wert für D-Malat beträgt 1,1 mmol/l (Tris, pH 8,5, 25 °C) bzw. 1,5 mmol/l (HEPES, pH 9,0, 25 °C). Der Km-Wert für $NAD^+$ beträgt 0,4 mmol/l (Tris, pH 8,5, 25 °C). Das erfindungsgemäße Enzym besitzt nach Reinigung eine spezifische Aktivität von mindestens 25 U/mg Protein. Durch Reinigung war es möglich, die Fremdaktivitäten in der Enzympopulation weitgehend zu entfernen:
z.B.

| | |
|---|---|
| L-Malat-Dehydrogenase | ≦0,01 %, |
| L-Lactat-Dehydrogenase | ≦0,01 %, |
| Glutamat-Dehydrogenase | ≦0,01 % |
| $\beta$-Galactose-Dehydrogenase | <0,01 % und |
| NADH-Oxidase | <0,01 %. |

Die Erfindung beinhaltet auch ein Verfahren zur Gewinnung des Enzyms durch Züchtung von E. coli Zellen in Gegenwart von Malat und Gewinnung des Enzyms aus diesen Zellen. Besonders bevorzugt wird E.coli DSM 5496 verwendet. Dabei werden die Zellen auf übliche Weise aufgeschlossen und der Rohextrakt durch Fällung mit polymerem Amin (Polymin-G20), Acetonfällung und Erwärmung auf 56 °C vorgereinigt. Das vorgereinigte Enzym wird weiterhin durch Anionenaustauscher-(DEAE-Sepharose)-Chromatographie, hydrophobe (Phenyl-Sepharose) Chromatographie, Phenylsepharose-Chromatographie, Ammoniumsulfatfällung und Dialyse gereinigt.

Ein weiterer Gegenstand der Erfindung ist ein Verfahren zur Stabilisierung einer NAD-abhängigen D-Malat-Dehydrogenase, decarboxylierend, bei dem eine Lösung des Enzyms mit einem Copolymer aus Saccharose und Epichlorhydrin (Ficoll), vorzugsweise Ficoll 70 (Molekulargewicht 70 kD), versetzt und dann lyophilisiert wird. Bevorzugt ist ein Verfahren, bei dem die Enzymlösung auf eine Konzentration von 200 U/ml eingestellt und mit 30 mg Ficoll 70 pro ml Enzymlösung versetzt wird. Das lyophilisierte Enzym besitzt selbst nach fünfwöchiger Lagerung bei 37 °C noch eine Restaktivität von mehr als 80 %.

Ein Gegenstand der Erfindung ist ebenfalls ein Verfahren zur Bestimmung von D-Malat, bei dem ein erfindungsgemäßes Enzym verwendet wird. Dabei wird die Umsetzung von D-Malat zu Pyruvat und $CO_2$ in Gegenwart von $NAD^+$ gemessen, das zu NADH und $H^+$ reduziert wird. Bevorzugt ist ein Verfahren, bei dem die Bildung von NADH photometrisch gemessen wird. Besonders bevorzugt ist ein Verfahren, bei dem die enzymatische Umsetzung von D-Malat mit einer Farbreaktion mit Tetrazoliumsalzen zur Erhöhung der Meßempfindlichkeit gekoppelt wird. Mit Hilfe des erfindungsgemäßen D-Malic-Enzyms ist es möglich, D-Malat-Konzentrationen auch bei ca. 250fachem Überschuß an L-Malat in Fruchtsäften und Wein zu bestimmen. Bei dem erfindungsgemäßen Verfahren ergeben sich nur vernachlässigbar geringe Störeinflüsse durch die Anwesenheit von Lebensmittel-Begleitstoffen.

Bei einem erfindungsgemäßen Verfahren zur D-Malat-Bestimmung können als Puffersubstanzen allgemein übliche, wie z.B. Tris, HEPES, Glycin, DEA und TES, verwendet werden. Besonders bevorzugt sind Tris oder/und HEPES. Die Messung laßt sich in einem pH-Bereich von 7,5 bis 10,0 ausführen. Bevorzugt ist dabei der pH-Bereich von 8,0 bis 9,5, am meisten bevorzugt der pH-Bereich von 8,8 bis 9,1.

Das erfindungsgemäße Verfahren wird bei einer Konzentration des Puffers in der Testlösung von 20 mmol/l bis 200 mmol/l durchgeführt. Bevorzugt ist dabei eine Konzentration des Puffers von 40 mmol/l bis 100 mmol/l, noch bevorzugter ist eine Konzentration des Puffers der Testlösung von 50 mmol/l.

Das erfindungsgemäße Verfahren zur D-Malat-Bestimmung kann in Gegenwart von $Mg^{2+}$- oder $Mn^{2+}$-Ionen durchgeführt werden. Obwohl $Mn^{2+}$-Ionen die Reaktion starker aktivieren, ist Einsatz von $Mg^{2+}$-Ionen bevorzugt, da in Gegenwart von $Mn^{2+}$ leicht eine Braunsteinbildung ($MnO_2$) beobachtet werden kann. Geeignet ist eine Konzentration der $Mg^{2+}$-Ionen in der Testlösung von 0,6 mmol/l bis 10 mmol/l. Bevorzugt ist eine Mgz.-Ionenkonzentration von 1,0 mmol/l bis 4,0 mmol/l, am meisten bevorzugt ist eine Konzentration von 3,3 mmol/l $Mg^{2+}$-Ionen in der Testlösung.

Das erfindungsgemäße Verfahren zur D-Malat-Bestimmung wird bei einer $NAD^+$-Konzentration von 0,3 mmol/l bis 5,0 mmol/l, vorzugsweise 0,6 mmol/l in der Testlösung durchgeführt.

Ein Gegenstand der Erfindung ist schließlich auch ein Reagenz zur D-Malat-Bestimmung, das erfindungsgemäße D-MDH, Puffer, $Mg^{2+}$-Ionen und $NAD^+$ enthält. Bevorzugt ist ein Reagenz, das Tris- und/oder HEPES-Puffer enthält. Besonders bevorzugt ist ein Reagenz, das 20 mmol/l bis 200 mmol/l Tris- oder/und HEPES-Puffer, pH 7,5 bis 10, 0,6 mmol/l bis 10 mmol/l $Mg^{2+}$-Ionen und 0,3 mmol/l bis 5,0 mmol/l $NAD^+$ enthält. Am meisten bevorzugt ist ein Reagenz, das 50 mmol/l Tris- oder/und HEPES-Puffer, 3,3 mmol/l

$Mg^{2+}$-Ionen und 0,6 mmol/l $NAD^+$ enthält.

Durch folgende Beispiele soll die Erfindung weiter verdeutlicht werden:

1. Fermentation, Anreicherung und Stabilisierung der D-MDH

Fermentation

Vorkulturen des E. coli Stammes DSM 5496 werden in LB-Medium (10 g Trypton, 5 g Hefeextrakt, 5 g NaCl auf 1000 ml Wasser, pH 7,0 bis 7,2) angezüchtet.

Bei Induzierung der D-MDH-Aktivität wird folgendes Medium verwendet:

7,1 g $Na_2HPO_4$,
20 g $K_2HPO_4$ x 3 $H_2O$,
0,25 g $MgSO_4$ x 7 $H_2O$,
2 g $(NH_4)_2SO_4$,
14 mg $CaCl_2$ x 2 $H_2O$,
4 g D,L-Malat
auf 1000 ml $H_2O$, pH 6,8 bis 7,0.

Nach Sterilisation werden diesem Medium folgende Supplemente beigemischt:

Vitamine:

| | |
|---|---|
| Thiamin HCL | 1 mg/l |
| p-Aminobenzoesäure | 0,2 mg/l |
| Pyridoxin | 0,2 mg/l |
| Riboflavin | 0,2 mg/l |
| Ca-Panthothenat | 1 mg/l |
| Folsäure | 0,2 mg/l |
| Biotin | 1 mg/l |

Spurenelemente:

| | |
|---|---|
| $MgCl_2$ x 4 $H_2O$ | 0,5 mg/l |
| $ZnSO_4$ x 7 $H_2O$ | 1 mg/l |
| $CuSO_4$ x 5 $H_2O$ | 0,5 mg/l |
| $CoCl_2$ x 6 $H_2O$ | 0,5 mg/l |
| $NiCl_2$ x 6 $H_2O$ | 0,5 mg/l |
| $Na_2MoO_4$ x 2 $H_2O$ | 0,05 mg/l |
| $FeCl_3$ x 6 $H_2O$ | 1,5 mg/l |

Die Kulturen werden bei 37 °C unter starker Belüftung angezüchtet. Die Ernte der Zellen erfolgt in der spätlogarithmischen Wachstumsphase. Anreicherung

Aufschluß:

700 g frische oder gefrorene E. coli DSM 5496 Zellen mit 4 Volumina kaltem 50 µmol/l Tris-HCl-Puffer, pH 7,5 suspendieren. 10 ml für eine Vorprobe zur Bestimmung der Enzymaktivität entnehmen. Bakteriensuspension mit Manton-Gaulin Hochdruckdispersion bei 600 bar zweimal aufschließen. Mit kaltem entionisiertem Wasser nachspülen.

Volumen (V) = 3,5 l

Gesamtaktivität = $4{,}5\times10^4$ U.

Polymin G-20-Abtrennung:

Nach Vorprobe den Rohextrakt mit 1 bis 3 % einer 10%igen Polymin G-20 Losung, pH 7,0 versetzen und 30 Minuten lang auf der Beckmann-Zentrifuge abzentrifugieren.

V = 3,5 l

Gesamtaktivität = $4{,}5\times10^4$ U

Acetonfällung:

Den blanken Polymin-Überstand mit 0,8 Volumen tiefgekühltem Aceton versetzen und 15 Minuten lang auf der Beckmann-Zentrifuge abzentrifugieren. Den Aceton-Niederschlag in 1 l Tris-HCl-Puffer, 50 µmol/l, pH 7,5, aufnehmen. Ca. eine Stunde im Eisbad rühren lassen, dann 20 Minuten lang auf der Beckmann-Zentrifuge abzentrifugieren.

V = 1 l

Gesamtaktivität = $4\times10^4$ U.

Erwärmung:

Nach Vorprobe (15 Minuten, 20 Minuten und 25 Minuten) wird die Hauptmenge des resuspendierten Aceton-Niederschlags im Wasserbad auf 56°C erhitzt. Der Gehalt der L-Malat-Dehydrogenase muß <1%, der Gehalt der Glutamat-Dehydrogenase <0,01% sein. Nach Erwärmung abkühlen und in Beckmann-Zentrifuge 20 Minuten zentrifugieren und über Glaswolle abfiltrieren.

V = 1 l

Gesamtaktivität = $3\times10^4$ U.

DEAE-Sepharose ff-Chromatographie:

Die Enzymlösung auf eine mit 50 µmol/l Tris-Puffer, pH 8,0 äquilibrierte DEAE-Sepharose ff-Säule aufziehen.

Kapazität: 200 bis 250 U/g Austauscher

Säulendimension: 3x20cm (ca. 130 g)

Waschung: 10 Säulenvolumina Tris-Puffer 50 µmol/l, pH 8,0; 0,11 mol/l NaCl

Elution: 10 Säulenvolumina Tris-Puffer 50 µmol/l, pH 8,0, 0,18 mol/l NaCl.

Alle aktiven Fraktionen vereinigen.

V = 250 ml

Gesamtaktivität = $2{,}1\times10^4$ U.

Der Gehalt an Glutamat-Dehydrogenase beträgt weniger als 0,01 %, der Gehalt an L-Malat-Dehydrogenase weniger als 0,01 % der Gesamtaktivität.

Phenyl-Sepharose ff-Chromatographie:

Die vereinigten aktiven DEAE-Sepharose-Fraktionen mit festem Ammoniumsulfat auf eine Konzentration von 1,0 mol/l Ammoniumsulfat einstellen. Die Enzymlösung auf eine mit Tris-Puffer 50 µmol/l, pH 7,5 und 1,0 mol/l Ammoniumsulfat äquilibrierte Phenylsepharose ff-Säule auftragen.

Kapazität: ca. 200 U/g Austauscher.

Säulendimension: 3x20 cm (ca. 100 g).

Waschung 10 Säulenvolumina Tris-Puffer 50 µmol/l, pH 7,5; 0,6 mol/l Ammoniumsulfat.

Elution: 10 Säulenvolumina Tris-Puffer 50 µmol/l, pH 7,5; 0,3 mol/l Ammoniumsulfat.

Die aktiven Fraktionen vereinigen.

V = 300 ml,

Gesamtaktivität = $1{,}5\times10^4$ U.

Der Gehalt an Lactat-Dehydrogenase beträgt weniger als 0,01 % der Gesamtaktivität.

Ammoniumsulfatfällung, Dialyse:

Vereinigte Eluate durch Zugabe von Ammoniumsulfat auf eine Konzentration von 2,5 mol/l Ammoniumsulfat einstellen. 30 Minuten lang in der Beckmann-Zentrifuge zentrifugieren und den Niederschlag konzentriert in 50 µmol/l Tris-Puffer, pH 7,5, aufnehmen und erschöpfend gegen diesen Puffer über Nacht bei 4°C dialysieren.

V = 75 ml,

Gesamtaktivität = $1{,}2\times10^4$ U.

Lyophilisation:

Das Dialysat mit Dialysepuffer auf eine Konzentration von 200 U D-MDH/ml einstellen und pro ml

Enzymlösung 30 ml Ficoll 70 (polymere Saccharose, mit Epichlorhydrin vernetzt, MG = 70.000), vorgelöst in $H_2O$ zugeben und lyophilisieren.
Ausbeute: 10.000 U D-MDH
spezifische Aktivität 25 U/mg Protein Gesamtmenge an Lyophilisat 2,3 g.
100 mg Lyophilisat enthalten 67 mg Ficoll 70, 15 mg Enzymprotein und 18 mg Tris-Puffer.

**Beispiel 2**

Bestimmung von D-Malat in reinen Lösungen

Das D-Malic-Enzym setzt D-Malat zu Pyruvat und $CO_2$ in Gegenwart von $NAD^+$ um, wobei aus $NAD^+$ NADH gebildet wird. Die NADH-Konzentration ist proportional zur Konzentration des umgesetzten D-Malats und kann photometrisch gemessen werden (Absorption bei 339 nm). Die Messung kann auch mit einer Hg-Lampe bei 365 nm oder 334 nm durchgeführt werden. Die Schichtdicke der Küvette beträgt 1 cm, die Meßtemperatur 20 bis 25 °C und das Testvolumen in der Küvette 3,01 ml.

D-Äpfelsäure-Bestimmung:

Wellenlänge: 339 nm, Hg 365 nm oder Hg 334 nm
Schichtdicke: 1 cm
Temperatur: 20 bis 25 °C
Testvolumen: 3,01 ml

| In Küvetten pipettieren | Leerwert | Probe | Konzentration im Test |
|---|---|---|---|
| HEPES-Puffer, pH 9,0 | 1,00 ml | 1,00 ml | 0,05 mol/l |
| $MgCl_2 \cdot 6H_2O$ | 0,10 ml | 0,10 ml | 3,27 mmol/l |
| NAD | 0,10 ml | 0,10 ml | 0,60 mmol/l |
| bidest. Wasser | 1,80 ml | 1,70 ml | |
| Probe- bzw. Standardlösung(D-Malat) | -- | 0,10 ml | bis ca. 130 µmol/l |
| mischen, nach ca. 5 Minuten $E_1$ messen. Reaktion starten durch Zugabe von | | | |
| D-MDH | 0,01 ml | 0,01 ml | 265 U/l |
| mischen, nach Ablauf der Reaktion (ca. 20 Minuten) $E_2$ messen. | | | |

Die Berechnung der Extinktion ΔE geschieht folgendermaßen:

$$\Delta E = (E_2 - E_1)_{Probe} - (E_2 - E_1)_{LW}$$

Die Konzentration von D-Malat ergibt sich wie folgt:

$$c = \frac{3{,}01 \cdot 134{,}09}{\epsilon \cdot 1 \cdot 0{,}1 \cdot 1000} \cdot \Delta E = [\text{g D-Äpfelsäure/l Probelösung}]$$

Dabei bedeutet $\epsilon$ der Extinktionskoeffizient von NADH bei:
339 nm = 6,3 (l/mmol • cm)
Hg 365 nm = 3,4 (l/mmol • cm)
Hg 334 nm = 6,18 (l/mmol • cm).

**Beispiel 3**

Bestimmung des D-Malat-Gehalts in Weißwein

10 ml einer Weinprobe werden mit KOH (5 mol/l) auf pH 7,0 eingestellt und mit Wasser auf 20 ml aufgefüllt. Der Inhalt wird gut gemischt und anschließend durch ein Faltenfilter filtriert. 1 ml klares Filtrat wird zum Test eingesetzt (siehe Beispiel 2).

**Patentansprüche**

1. $NAD^+$-abhängige D-Malat-Dehydrogenase, decarboxylierend (E.C. 1.1.1.83) aus E. coli,
**dadurch gekennzeichnet,**
daß für die Enzymaktivität keine Anwesenheit von $K^+$, $Rb^+$ oder $NH_4{}^+$-Ionen erforderlich ist, daß seine Aktivität nicht durch EDTA (0,7 mg/ml Testlösung) gehemmt wird, daß die Umsetzung von D-Malat zu Pyruvat und $CO_2$ bei Anwesenheit von $NADP^+$ weniger als 0,5 % der Umsetzung bei Anwesenheit von $NAD^+$ beträgt, daß es aus zwei Untereinheiten mit jeweils etwa 42 kD Molekulargewicht (SDS-Gel) besteht und daß es aus E.coli DSM 5496 erhältlich ist.

2. Enzym nach Anspruch 1,
**dadurch gekennzeichnet,**
daß es nach Reinigung eine spezifische Aktivität von mindestens 25 U/mg Protein besitzt.

3. Verfahren zur Gewinnung des Enzyms nach einem der Ansprüche 1 oder 2, **dadurch gekennzeichnet**, daß man E. coli Zellen in Gegenwart von Malat züchtet und das Enzym aus den Zellen gewinnt.

4. Verfahren zur Stabilisierung einer NAD-abhängigen D-Malat-Dehydrogenase, decarboxylierend gemäß Anspruch 1 oder 2,
**dadurch gekennzeichnet,**
daß eine Lösung des Enzyms mit einem Copolymer aus Saccharose und Epichlorhydrin, vorzugsweise mit einem Molekulargewicht von etwa 70 kD versetzt und lyophilisiert wird.

5. Verfahren zur D-Malat-Bestimmung mit einer D-Malat-Dehydrogenase gemäß Anspruch 1 oder 2,
**dadurch gekennzeichnet,**
daß als D-Malat-Dehydrogenase ein Enzym nach einem der vorhergehenden Ansprüche verwendet wird.

6. Reagenz zur D-Malat-Bestimmung,
**dadurch gekennzeichnet,**
daß es D-Malat-Dehydrogenase nach einem der Ansprüche 1 oder 2, Puffer, $Mg^{2+}$-Ionen und $NAD^+$ enthält.

7. Reagenz nach Anspruch 6,
**dadurch gekennzeichnet,**

daß es 20 mmol/l bis 200 mmol/l Tris- oder/und HEPES-Puffer, pH 7,5 bis 10, 0,6 mmol/l bis 10 mmol/l $Mg^{2+}$-Ionen und 0,3 mmol/l bis 5,0 mmol/l $NAD^+$ enthält.

**Claims**

1. $NAD^+$-dependent D-malate dehydrogenase, decarboxylating (E.C. 1.1.1.83) from E. coli,
**wherein**
the presence of $K^+$, $Rb^+$ or $NH_4^+$ ions is not necessary for the enzyme activity, its activity is not inhibited by EDTA (0.7 mg/ml test solution), the conversion of D-malate to pyruvate and $CO_2$ in the presence of $NADP^+$ is less than 0.5 % of the conversion in the presence of $NAD^+$, it consists of two subunits which each have a molecular weight of about 42 kD (SDS gel) and it is obtainable from E. coli DSM 5496.

2. Enzyme as claimed in claim 1,
**wherein**
it has a specific activity of at least 25 U/mg protein after purification.

3. Process for the isolation of the enzyme as claimed in one of the claims 1 or 2,
**wherein**
E. coli cells are cultured in the presence of malate and the enzyme is isolated from the cells.

4. Process for the stabilization of an HAD-dependent D-malate dehydrogenase, decarboxylating as claimed in claim 1 or 2,
**wherein**
a copolymer of sucrose and epichlorohydrin preferably with a molecular weight of about 70 kD is added to a solution of the enzyme and it is lyophilized.

5. Method for the determination of D-malate with a D-malate dehydrogenase as claimed in claim 1 or 2,
**wherein**
an enzyme as claimed in one of the previous claims is used as the D-malate dehydrogenase.

6. Reagent for the determination of D-malate,
**wherein**
it contains D-malate dehydrogenase as claimed in one of the claims 1 or 2, buffer, $Mg^{2+}$ ions and $NAD^+$.

7. Reagent as claimed in claim 6,
**wherein**
it contains 20 mmol/l to 200 mmol/l Tris or/and HEPES buffer, pH 7.5 to 10, 0.6 mmol/l to 10 mmol/l $Mg^{2+}$ ions and 0.3 mmol/l to 5.0 mmol/l $NAD^+$.

**Revendications**

1. D-malate-déhydrogénase de dépendance $NAD^+$, décarboxylant, (E.C. 1.1.1.83) à partir d'E. coli, caractérisé en ce que pour l'activité enzymatique la présence de $K^+$, $Rb^+$ ou d'ions $NH_4^+$ n'est pas indispensable, en ce que son activité n'est pas inhibée par EDTA (0,7 mg/ml solution de test), en ce que la conversion de D-malate en pyruvate et $CO_2$ en présence de $NADP^+$ est inférieure à 0,5 % de la conversion en présence de $NAD^+$, en ce qu'elle est constituée par deux sous-unités avec chaque fois environ 42 kD de masse moléculaire (gel SDS) et en ce qu'elle peut être obtenue à partir de E. coli DSM 5496.

2. Enzyme selon la revendication 1, caractérisée en ce qu'elle possède après purification une activité spécifique d'au moins 25 U/mg de protéine.

3. Procédé pour l'obtention de l'enzyme selon l'une des revendications 1 ou 2, caractérisé en ce que l'on cultive des cellules d'E. coli en présence de malate et en ce que l'on obtient l'enzyme à partir des cellules.

**4.** Procédé pour la stabilisation d'une D-malatedéhydrogénase dépendance NAD, décarboxylant, selon la revendication 1 ou 2, caractérisé en ce que l'on ajoute à une solution de l'enzyme un copolymère à partir de saccharose et d'épichlorhydrine, de préférence d'une masse moléculaire d'environ 70 kD et lyopholisé.

**5.** Procédé pour la détermination de D-malate avec une D-malate-déhydrogénase selon la revendication 1 ou 2, caractérisé en ce qu'en tant que D-malate-déhydrogénase, on utilise une enzyme selon l'une des revendications précédentes.

**6.** Réactif pour la détermination de D-malate caractérisé en ce qu'il contient de la D-malate-déhydrogénase selon l'une des revendications 1 ou 2, un tampon, des ions $Mg^{2+}$ et $NAD^+$.

**7.** réactif selon la revendication 6, caractérisé en ce qu'il contient 20 mmoles/l jusqu'à 200 mmoles/l de tampon Tris ou/et HEPES, pH de 7,5 jusqu'à 10, 0,6 mmole/l jusqu'à 10 mmoles/l d'ions $Mg^{2+}$ et 0,3 mmole/l jusqu'à 5,0 mmoles/l de $NAD^+$.